(19) Europäisches Patentamt
European Patent Office
Office européen des brevets

(11) **EP 4 703 880 A1**

(12) **EUROPEAN PATENT APPLICATION**
published in accordance with Art. 153(4) EPC

(43) Date of publication:
**04.03.2026 Bulletin 2026/10**

(21) Application number: **24796169.1**

(22) Date of filing: **25.04.2024**

(51) International Patent Classification (IPC):
**G06F 9/50** (2006.01)    **A61N 5/10** (2006.01)

(52) Cooperative Patent Classification (CPC):
**G06F 9/50; G06F 30/25; A61N 5/10**

(86) International application number:
**PCT/CN2024/089786**

(87) International publication number:
**WO 2024/222793 (31.10.2024 Gazette 2024/44)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.04.2023 CN 202310469281**

(71) Applicant: **Neuboron Therapy System Ltd.**
**Xiamen, Fujian 361026 (CN)**

(72) Inventors:
• **ZHONG, Wan-bing**
**Nanjing, Jiangsu 211112 (CN)**
• **CHEN, Jiang**
**Nanjing, Jiangsu 211112 (CN)**

(74) Representative: **Gunzelmann, Rainer**
**Wuesthoff & Wuesthoff**
**Patentanwälte und Rechtsanwalt PartG mbB**
**Schweigerstraße 2**
**81541 München (DE)**

(54) **CONTROL METHOD AND APPARATUS FOR DOSE CALCULATION SYSTEM, AND DOSE CALCULATION SYSTEM**

(57)    The present application relates to a control method and apparatus for a dose calculation system, and a dose calculation system. The method includes: building a dose calculation geometric model; determining a CPU and GPU joint dose calculation mode from a computing speed of a CPU and a computing speed of a GPU; and performing, by the CPU and the GPU, particle simulation according to the CPU and GPU joint dose calculation mode and the dose calculation geometric model to obtain a dose calculation result. By using this method, computing resources of the dose calculation system can be fully used, thereby maximizing the improvement of a dose calculation speed.

| | |
|---|---|
| Build a dose calculation geometric model | 102 |
| Determine a CPU and GPU joint dose calculation mode from a computing speed of a CPU and a computing speed of a GPU | 104 |
| Perform, by the CPU and the GPU, particle simulation according to the CPU and GPU joint dose calculation mode and the dose calculation geometric model to obtain a dose calculation result | 106 |

Figure 1

## Description

### TECHNICAL FIELD

The present application relates to the technical field of computers, and in particular to a control method and apparatus for a dose calculation system, and a dose calculation system.

### BACKGROUND

With the development of atomic science, radiation therapy such as cobalt-60 therapy, linear accelerator therapy and electron beam therapy has become one of the main means for cancer treatment. However, conventional photon or electron therapy is limited by the physical properties of radiation itself and may cause damages to a large number of normal tissues in a beam path while killing tumor cells. In addition, due to differences in the sensitivity of the tumor cells to the radiation, the efficacy of conventional radiation therapy is often unsatisfactory for malignant tumors with higher radioresistance (e.g., glioblastoma multiforme and melanoma).

To reduce radiation damages to normal tissues around a tumor, the concept of targeted therapy from chemotherapy has been applied to radiation therapy. For the tumor cells with high radioresistance, currently, radiation sources with high relative biological effectiveness (RBE), such as proton therapy, heavy particle therapy, and neutron capture therapy, are actively developed. The neutron capture therapy combines both of the concepts mentioned above. For example, boron neutron capture therapy (BNCT) utilizes the specific accumulation of boron-containing drugs in tumor cells, in combination with precise beam control, to offer a superior cancer treatment option over the conventional radiation therapy.

In the boron neutron capture therapy, the property of a high capture cross-section of a boron-10 ($^{10}B$)-containing drug for thermal neutrons is used. By means of $^{10}B(n,\alpha)^{7}Li$ neutron capture and nuclear fission reaction, two heavily charged particles, $^{4}He$ and $^{7}Li$, are generated. The total range of these two particles is approximately equal to the size of one cell, and thus the radiation damage caused to an organism can be confined to the cellular level. When the boron-containing drug selectively accumulates in the tumor cells and is combined with an appropriate neutron source, the purpose of locally killing the tumor cells can be achieved without causing significant damages to normal tissues.

To maximize the killing of cancer cells by radiation particles while minimizing damage to normal cells, imaging scans such as CT or PET are typically performed on a living organism prior to treatment. Based on the scan results, tissue material information of the living organism is obtained. A computational model is then established based on the material information and a radiation source to simulate the transport process of radiation particles within the living organism. Finally, the dose distribution of the radiation particles in the living organism is obtained, and a plan with an optimal dose distribution for the living organism is selected as a treatment plan for the living organism.

Currently, the most accurate and reliable method for dose calculation is the Monte Carlo method. However, the Monte Carlo method requires a long computation time, making it necessary to improve a dose calculation speed. In common technologies, the dose calculation speed is improved from the perspective of computer hardware, specifically by accelerating dose calculation using a single type of computer hardware of a dose calculation system. However, the dose calculation system includes a plurality of types of computer hardware. The use of only a single type of computer hardware for dose calculation leads to the waste of computing resources and fails to maximize the improvement of the dose calculation speed.

### SUMMARY

Based on this, it is necessary to address the aforementioned technical problems by providing a control method and apparatus for a dose calculation system, and a dose calculation system that allow for full use of computing resources of the dose calculation system to maximize the improvement of a dose calculation speed.

In a first aspect, the present application provides a control method for a dose calculation system including a CPU and a GPU, the method including:

> building a dose calculation geometric model;
> determining a CPU and GPU joint dose calculation mode from a computing speed of the CPU and a computing speed of the GPU; and
> performing, by the CPU and the GPU, particle simulation according to the CPU and GPU joint dose calculation mode and the dose calculation geometric model to obtain a dose calculation result.

The use of the CPU and the GPU for joint dose calculation allows for full use of the computing resources of the dose calculation system to maximize the improvement of the dose calculation speed.

In one embodiment, performing, by the CPU and the GPU, particle simulation according to the CPU and GPU joint dose calculation mode and the dose calculation geometric model to obtain the dose calculation result includes:

> when the CPU and GPU joint dose calculation mode is a first joint dose calculation mode,
> determining a respective number of particles to be simulated by the CPU and the GPU from the computing speed of the CPU, the computing speed of the GPU, and a total number of particles to be simulated;
> simulating, respectively by the CPU and the GPU, the respective number of particles to be simulated according to the dose calculation geometric model to obtain a CPU dose calculation result and a GPU dose calculation result; and

summing the CPU dose calculation result and the GPU dose calculation result to obtain the dose calculation result.

In one embodiment, determining the respective number of particles to be simulated by the CPU and the GPU from the computing speed of the CPU, the computing speed of the GPU, and the total number of particles to be simulated includes:

determining a computing speed proportion of the CPU and a computing speed proportion of the GPU from the computing speed of the CPU and the computing speed of the GPU;

determining the number of particles to be simulated by the CPU from the computing speed proportion of the CPU and the total number of particles to be simulated; and

determining the number of particles to be simulated by the CPU from the computing speed proportion of the GPU and the total number of particles to be simulated.

In one embodiment, performing, by the CPU and the GPU, particle simulation according to the CPU and GPU joint dose calculation mode and the dose calculation geometric model to obtain the dose calculation result further includes:

when the CPU and GPU joint dose calculation mode is a second joint dose calculation mode, performing, by the GPU, particle simulation according to the dose calculation geometric model;

when the GPU identifies that a number of colliding particles during the simulation exceeds a preset colliding particle threshold, performing, by the CPU, collision processing on the colliding particles to obtain a collision processing result; and

resuming, by the GPU, the particle simulation based on the collision processing result to obtain the dose calculation result.

In one embodiment, performing, by the GPU, particle simulation according to the dose calculation geometric model includes:
simulating, by the GPU, a particle transport process based on the dose calculation geometric model.
In one embodiment, the method further includes:

determining, by the GPU, cross-section information of a grid where a particle is located, and
determining whether the particle will collide during the transport process based on the cross-section information and a random sampling value; and

if a collision will occur, marking, by the GPU, the particle as a colliding particle.

In one embodiment, when the GPU identifies that the number of colliding particles during the simulation exceeds the preset colliding particle threshold, performing, by the CPU, collision processing on the colliding particles includes:

when the GPU identifies that the number of colliding particles during the simulation exceeds the preset colliding particle threshold, copying particle information of the colliding particles to the CPU; and
performing, by the CPU, collision processing on the colliding particles based on the particle information.

In one embodiment, determining the CPU and GPU joint dose calculation mode from the computing speed of the CPU and the computing speed of the GPU includes:

determining the CPU and GPU joint dose calculation mode to be a first joint dose calculation mode when a ratio of the computing speed of the CPU to the computing speed of the GPU is greater than a first threshold; and
determining the CPU and GPU joint dose calculation mode to be a second joint dose calculation mode when the ratio of the computing speed of the CPU to the computing speed of the GPU is less than a second threshold; where the first threshold is greater than the second threshold.

In one embodiment, determining the CPU and GPU joint dose calculation mode from the computing speed of the CPU and the computing speed of the GPU further includes:

when the ratio of the computing speed of the CPU to the computing speed of the GPU is less than or equal to the first threshold and greater than or equal to the second threshold,
determining the CPU and GPU joint dose calculation mode to be either the first joint dose calculation mode or the second joint dose calculation mode.

In one embodiment, the first threshold is 0.3, and the second threshold is 0.15.
In a second aspect, the present application further provides a control apparatus for a dose calculation system including a CPU and a GPU, the apparatus including:

a model building module configured to build a dose calculation geometric model;
a mode determination module configured to determine a CPU and GPU joint dose calculation mode from a computing speed of a CPU and a computing speed of a GPU;
a dose calculation module configured to control the CPU and the GPU to perform particle simulation according to the CPU and GPU joint dose calculation mode and the dose calculation geometric model to obtain a dose calculation result.

In one embodiment, the dose calculation module is further configured to: when the CPU and GPU joint dose calculation mode is a first joint dose calculation mode, determine a respective number of particles to be simulated by the CPU and the GPU from the computing speed of the CPU, the computing speed of the GPU, and a total number of particles to be simulated; control the CPU to simulate the respective number of particles to be simulated according to the dose calculation geometric model to obtain a CPU dose calculation result; control the GPU to simulate the respective number of particles to be simulated according to the dose calculation geometric model to obtain a GPU dose calculation result; and sum the CPU dose calculation result and the GPU dose calculation result to obtain the dose calculation result.

In one embodiment, the dose calculation module is further configured to: determine a computing speed proportion of the CPU and a computing speed proportion of the GPU from the computing speed of the CPU and the computing speed of the GPU; determine the number of particles to be simulated by the CPU from the computing speed proportion of the CPU and the total number of particles to be simulated; and determine the number of particles to be simulated by the CPU from the computing speed proportion of the GPU and the total number of particles to be simulated.

In one embodiment, the dose calculation module is further configured to: when the CPU and GPU joint dose calculation mode is a second joint dose calculation mode, control the GPU to perform particle simulation according to the dose calculation geometric model; when the GPU identifies that a number of colliding particles during the simulation exceeds a preset colliding particle threshold, control the CPU to perform collision processing on the colliding particles to obtain a collision processing result; and control the GPU to resume the particle simulation based on the collision processing result to obtain the dose calculation result.

In one embodiment, the dose calculation module is further configured to control the GPU to simulate a particle transport process based on the dose calculation geometric model.

In one embodiment, the dose calculation module is further configured to: control the GPU to determine cross-section information of a grid where a particle is located, and determine whether the particle will collide during the transport process based on the cross-section information and a random sampling value; and if a collision will occur, control the GPU to mark the particle as a colliding particle.

In one embodiment, the dose calculation module is further configured to: when the GPU identifies that the number of colliding particles during the simulation exceeds the preset colliding particle threshold, control the GPU to copy particle information of the colliding particles to the CPU; and control the CPU to perform collision processing on the colliding particles based on the particle information.

In one embodiment, the mode determination module is further configured to:
determine the CPU and GPU joint dose calculation mode to be the first joint dose calculation mode when a ratio of the computing speed of the CPU to the computing speed of the GPU is greater than a first threshold; and determine the CPU and GPU joint dose calculation mode to be the second joint dose calculation mode when the ratio of the computing speed of the CPU to the computing speed of the GPU is less than a second threshold; where the first threshold is greater than the second threshold.

In one embodiment, the mode determination module is further configured to: when the ratio of the computing speed of the CPU to the computing speed of the GPU is less than or equal to the first threshold and greater than or equal to the second threshold, determine the CPU and GPU joint dose calculation mode to be either the first joint dose calculation mode or the second joint dose calculation mode.

In one embodiment, the first threshold is 0.3, and the second threshold is 0.15.

In a third aspect, the present application further provides a dose calculation system. The system includes:

a model building module configured to build a dose calculation geometric model;
a mode determination module configured to determine a CPU and GPU joint dose calculation mode from a computing speed of a CPU and a computing speed of a GPU;
a CPU module configured to perform at least part of dose calculation according to the CPU and GPU joint dose calculation mode and the dose calculation geometric model; and
a GPU module configured to perform at least part of the dose calculation according to the CPU and GPU joint dose calculation mode and the dose calculation geometric model.

In a fourth aspect, the present application further provides a computer device. The computer device includes a memory and a processor. The memory stores a computer program, and the processor, when executing the computer program, causes the following steps to be implemented:

building a dose calculation geometric model;
determining a CPU and GPU joint dose calculation mode from a computing speed of a CPU and a computing speed of a GPU; and

performing, by the CPU and the GPU, particle simulation according to the CPU and GPU joint dose calculation mode and the dose calculation geometric model to obtain a dose calculation result.

In a fifth aspect, the present application further provides a computer-readable storage medium. The computer-readable storage medium has a computer program stored thereon, and the computer program, when executed by a processor, causes the following steps to be implemented:

building a dose calculation geometric model;
determining a CPU and GPU joint dose calculation mode from a computing speed of a CPU and a computing speed of a GPU; and
performing, by the CPU and the GPU, particle simulation according to the CPU and GPU joint dose calculation mode and the dose calculation geometric model to obtain a dose calculation result.

In a sixth aspect, the present application further provides a computer program product. The computer program product includes a computer program which, when executed by a processor, causes the following steps to be implemented:

building a dose calculation geometric model;
determining a CPU and GPU joint dose calculation mode from a computing speed of a CPU and a computing speed of a GPU; and
performing, by the CPU and the GPU, particle simulation according to the CPU and GPU joint dose calculation mode and the dose calculation geometric model to obtain a dose calculation result.

In the control method and apparatus for a dose calculation system, and the dose calculation system as described above, the introduction of a CPU and GPU joint acceleration mode in which the dose calculation geometric model is built, and the CPU and GPU joint dose calculation mode is then determined from the computing speeds of the CPU and the GPU, that is, a suitable joint dose calculation mode is selected based on the performances of the CPU and the GPU, allows for full use of computing resources, thereby maximizing the improvement of the dose calculation speed and reducing the dose calculation time. In addition, compared with using only the CPU or only the GPU for calculation, this method not only achieves higher computing efficiency but also maximizes the use of computing resources.

## BRIEF DESCRIPTION OF THE DRAWINGS

FIG. 1 is a schematic flowchart of a control method for a dose calculation system according to an embodiment;
FIG. 2 is a schematic flowchart of particle simulation by a CPU and a GPU according to a CPU and GPU joint dose calculation mode and a dose calculation geometric model according to an embodiment;
FIG. 3 is a schematic flowchart of particle simulation by a CPU and a GPU according to a CPU and GPU joint dose calculation mode and a dose calculation geometric model according to another embodiment;
FIG. 4 is a schematic plan view of a particle in a three-dimensional voxel tissue model when a boundary distance is greater than a collision distance according to an embodiment;
FIG. 5 is a schematic plan view of a particle in a three-dimensional voxel tissue model when a boundary distance is less than a collision distance according to an embodiment;
FIG. 6 is a schematic flowchart of determining a CPU and GPU joint dose calculation mode according to an embodiment;
FIG. 7 is structural block diagram of a control apparatus for a dose calculation system according to an embodiment;
FIG. 8 is a structural block diagram of a dose calculation system according to an embodiment; and
FIG. 9 is a diagram of an internal structure of a computer device according to an embodiment.

## DETAILED DESCRIPTION OF THE EMBODIMENTS

In order to make the objective, technical solutions, and advantages of the present application clearer, the present application is further described in detail below with reference to the accompanying drawings and the embodiments. It should be understood that the specific embodiments described herein are merely used to explain the present application and are not intended to limit the present application.

Radiation therapy is an important means for cancer treatment. Before radiation therapy, a radiation treatment plan needs to be developed, and dose calculation is one of the core contents of the radiation treatment plan. Currently, the most accurate and reliable method for dose calculation is the Monte Carlo method. The Monte Carlo method can simulate the transport trajectory of each particle, thereby precisely calculating the dose distribution inside a target subject. However, the Monte Carlo method has a relatively slow convergence speed, and a large number of particles need to be simulated in order to obtain a reliable dose calculation result, which leads to a long computation time. Therefore, there is a need to improve a dose calculation speed.

Optimizations for the Monte Carlo method mainly focus on two directions: one is to increase the convergence speed to reduce the number of particles that need to be simulated; and the other is direct acceleration, so that less time is required to simulate the same number of particles. Direct acceleration is mainly to improve the dose calculation speed from the perspective of computer hardware. The computer hardware of a dose calculation system mainly includes a CPU (Center Processing Unit) and a GPU (Graphic Processing Unit). Currently, most dose calculation systems use multi-core CPUs to accelerate dose calculation, and some dose calculation systems use GPUs to accelerate dose calculation. Specifically, for dose calculation in photon beam therapy and proton therapy, the use of GPUs results in significant acceleration effects and is substantially better than multi-core CPUs at the same price. For dose calculation in the boron neutron capture therapy, since the simulation process of neutrons is much more complex than that of photons, protons, etc., there is currently no corresponding dose calculation system that uses GPU acceleration methods for dose calculation. Instead, multi-core CPUs are still used to accelerate dose calculation. Therefore, conventional technologies all rely on a single type of computer hardware of the dose calculation system to accelerate dose calculation.

However, most dose calculation systems are equipped with both multi-core CPUs and GPUs. Whether using only CPUs or only GPUs to accelerate the dose calculation process will result in the waste of the other type of computing resources. To fully use the computing resources of the dose calculation system, a control method for a dose calculation system that can use both a CPU and a GPU to accelerate dose calculation is provided.

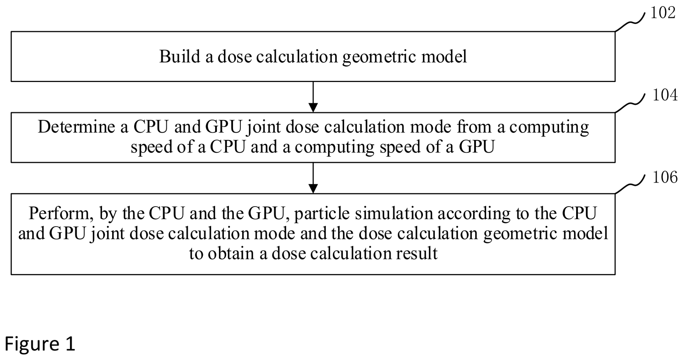

In an embodiment, as shown in FIG. 1, a control method for a dose calculation system is provided. This embodiment is exemplified by applying the method to a dose calculation system. In this embodiment, the method includes the following steps.

In step 102, a dose calculation geometric model is built.

The dose calculation geometric model refers to a geometric model which is used for particle simulation during dose calculation. A particle may be at least one of a neutron, a photon, a proton, or an electron.

The dose calculation system builds the dose calculation geometric model. The dose calculation geometric model may include a radiation source model, a three-dimensional voxel tissue model, a shield model, a carrying apparatus model, etc. The radiation source model may be a collimator model, a beam exit model of an irradiation apparatus, or the like, which contains information such as the energy, position and orientation of a radiation source. The three-dimensional voxel tissue model is built from medical images of a target subject, and refers to a three-dimensional model containing tissue information of the target subject. The tissue information may include tissue type and tissue density. The shield model refers to information about a shielding material, etc., placed on a skin surface of the target subject or between the radiation source and the target subject, and is configured to shield normal tissues of the target subject from a radiation beam emitted by the radiation source model. The carrying apparatus model refers to a carrying apparatus for carrying out radiation therapy on the target subject, such as a treatment couch or a treatment chair. It should be understood that in most dose calculations, the dose calculation geometric model may include only the radiation source model and the three-dimensional voxel tissue model.

Further, the dose calculation system can perform three-dimensional reconstruction on the medical images to obtain a three-dimensional model, perform three-dimensional meshing on the three-dimensional model to obtain a three-dimensional voxel model, outline at least one region of interest (ROI) according to a predefined ROI boundary, and determine the tissue type and tissue density in the POI region, thereby obtaining a three-dimensional voxel tissue model containing the tissue information of the target subject. The medical images may be X-ray images, CT images, etc., and the medical images may be images of a part or a plurality of parts. Specifically, the medical images of the target subject may be obtained by scanning one or more body parts of the target subject in advance by a scanning device, or by emitting X-rays onto one or more body parts of the subject to obtain scanning data or projection data of the subject and performing image reconstruction on the scanning data or projection data. Optionally, the medical images may also be pre-stored in a terminal, and the medical images sent by the terminal are acquired during dose calculation.

In step 104, a CPU and GPU joint dose calculation mode is determined from a computing speed of the CPU and a computing speed of the GPU.

The CPU and the GPU are two independent modules of the dose calculation system, and the GPU operates according to instructions sent by the CPU. The computing speed of the CPU and the computing speed of the GPU respectively refer to the number of particles that the CPU and the GPU can simulate per unit time.

The computing speeds of the CPU and the GPU are obtained by pre-simulating a small number of particles on the CPU and the GPU. Thus, a computing capability of the CPU can be determined based on a ratio relationship between the computing speeds of the CPU and the GPU, and the CPU and GPU joint dose calculation mode can thus be determined based on the computing capability of the CPU. The CPU and GPU joint dose calculation mode is used to determine collaboration modes of the CPU and the GPU in the dose calculation process. The use of the CPU and the GPU for joint dose calculation allows for full use of the computing resources of the dose calculation system to maximize the improvement of the dose calculation speed. When the ratio relationship between the computing speeds of the CPU and the GPU changes, the determined CPU and GPU joint dose calculation mode may also vary. Of course, the computing speeds of the GPU and the CPU may also be evaluated by other methods known to those skilled in the art.

Further, two preset CPU and GPU joint dose calculation modes may be included: one consists in that the CPU and the GPU independently complete the particle simulation process, and the other consists in that the CPU and the GPU separately complete part of the particle simulation process. By determining the computing capability of the CPU, one joint dose calculation mode is selected from the preset CPU and GPU joint dose calculation modes as the CPU and GPU joint dose calculation mode.

In step 106, particle simulation is performed by the CPU and the GPU according to the CPU and GPU joint dose calculation mode and the dose calculation geometric model to obtain a dose calculation result.

The CPU and the GPU, according to their respective collaboration modes in the dose calculation process determined in the CPU and GPU joint dose calculation mode, then use the Monte Carlo method to simulate a particle transport process based on the dose calculation geometric model, to obtain the dose calculation result.

In this embodiment, the introduction of a CPU and GPU joint acceleration mode in which the dose calculation geometric model is built, and the CPU and GPU joint dose calculation mode is then determined from the computing speeds of the CPU and the GPU, that is, a suitable joint dose calculation mode is selected based on the performances of the CPU and the GPU, allows for full use of computing resources, thereby maximizing the improvement of the dose calculation speed and reducing the dose calculation time. In addition, compared with using only the CPU or only the GPU for calculation, this method not only achieves higher computing efficiency but also maximizes the use of computing resources.

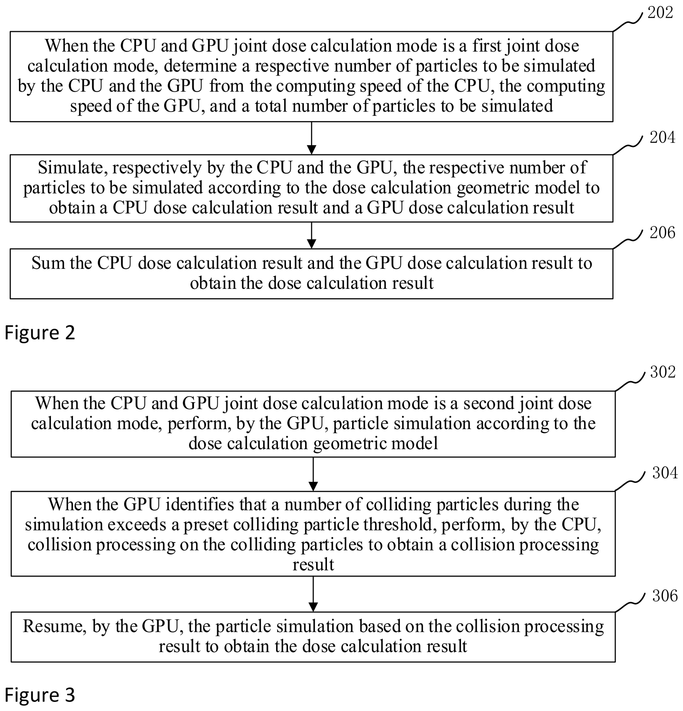

Since there are various preset CPU and GPU joint dose calculation modes, it is necessary to select one joint dose calculation mode from the preset CPU and GPU joint dose calculation modes as the CPU and GPU joint dose calculation mode. In an embodiment, as shown in FIG. 2, performing, by the CPU and the GPU, particle simulation according to the CPU and GPU joint dose calculation mode and the dose calculation geometric model to obtain the dose calculation result includes:

> step 202: when the CPU and GPU joint dose calculation mode is a first joint dose calculation mode, determining a respective number of particles to be simulated by the CPU and the GPU from the computing speed of the CPU, the computing speed of the GPU, and a total number of particles to be simulated;
> step 204: simulating, respectively by the CPU and the GPU, the respective number of particles to be simulated according to the dose calculation geometric model to obtain a CPU dose calculation result and a GPU dose calculation result; and
> step 206: summing the CPU dose calculation result and the GPU dose calculation result to obtain the dose calculation result.

The first joint dose calculation mode refers to a mode where both the CPU and the GPU need to independently complete the particle simulation process. The total number of particles to be simulated refers to the total number of particles that the dose calculation system needs to simulate during dose calculation.

When the dose calculation is accelerated for the Monte Carlo method, an acceleration method called particle parallelism is typically used. For both a multi-core CPU and a GPU, the principles of acceleration lie in allocating computing tasks to a plurality of computing cores, thereby increasing computing speed. For a specific model of CPU or GPU, the computing capability of each core of the CPU or the GPU is identical. Therefore, computing loads may be allocated simply by equal allocation based on the number of computing cores. However, considering that there are differences in computing capabilities between CPUs and GPUs, and the computing capabilities of different models of CPUs and GPUs vary significantly, to achieve a good acceleration effect, it is necessary to ensure that the computing loads on the CPU and the GPU are proportional to their respective computing capabilities. This enables the CPU and GPU to complete dose calculation almost simultaneously, thereby minimizing the waiting time as much as possible.

When the computing capability of the CPU is determined to be high or medium based on the ratio relationship between the computing speeds of the CPU and the GPU, the CPU and GPU joint dose calculation mode is determined to be the first joint dose calculation mode. Specifically, the total number of particles to be simulated is obtained according to the first joint dose calculation mode, and the computing loads are allocated to the CPU and the GPU based on the computing speed of the CPU, the computing speed of the GPU, and the total number of particles to be simulated, to obtain the number of particles to be simulated by the CPU and the number of particles to be simulated by the GPU. The sum of the number of particles to be simulated by the CPU and the number of particles to be simulated by the GPU equals the total number of particles to be simulated. Thus, the CPU simulates a respective number of particles to be simulated by the CPU based on the dose calculation geometric model according to the Monte Carlo method, to obtain the CPU dose calculation result. Also, the GPU simulates a respective number of particles to be simulated by the GPU based on the dose calculation geometric model according to the Monte Carlo method, to obtain the GPU dose calculation result, and transmits the GPU dose calculation result to the CPU. Then, the CPU adds the CPU dose calculation result to the GPU dose calculation result to obtain the dose calculation result.

Optionally, the CPU is a multi-core CPU, and the multi-core CPU is used for parallel particle simulation. The particle simulation process by the multi-core CPU is as follows. The number of processes or threads of the CPU is obtained to obtain a value n, and the respective number of particles to be simulated by the CPU are then equally divided into n parts. Thereafter, each process or thread simulates and counts each part of the particles based on the dose calculation geometric model according to the Monte Carlo method, and the counts obtained by each process or thread are then added to obtain the CPU dose calculation result.

Optionally, the parallel computing of the GPU is implemented by using multi-processors of the GPU. The simulation and calculation process of the GPU is as follows. Cross-section information, random sampling values, etc. of the respective number of particles to be simulated by the GPU are transferred from a CPU memory to a GPU memory. Then, a single particle is simulated, calculated and counted by each processor of the GPU based on the dose calculation geometric model according to the Monte Carlo method, and the count results are added into a global count. Thereafter, it is determined whether there are any unsimulated particles. If not, the count result, i.e., the GPU dose calculation result, is transferred from the GPU memory to the CPU memory. If there are still unsimulated particles, the process returns to the previous step to resume simulating and counting the unsimulated particles until all particles have been simulated, to obtain the GPU dose calculation result.

In this embodiment, when the computing capability of the CPU is high or medium, the CPU and GPU respectively calculate their respective number of particles based on their computing capabilities, which achieves higher efficiency compared with using only the CPU or only the GPU for calculation.

In an optional implementation of the above embodiment, determining the respective number of particles to be simulated by the CPU and the GPU from the computing speed of the CPU, the computing speed of the GPU, and the total number of particles to be simulated includes: determining a computing speed proportion of the CPU and a computing speed proportion of the GPU from the computing speed of the CPU and the computing speed of the GPU; determining the number of particles to be simulated by the CPU from the computing speed proportion of the CPU and the total number of particles to be simulated; and determining the number of particles to be simulated by the CPU from the computing speed proportion of the GPU and the total number of particles to be simulated.

The computing speed proportion of the CPU refers to the proportion of the computing speed of the CPU in the sum of the computing speeds of the CPU and the GPU. The computing speed proportion of the GPU refers to the proportion of the computing speed of the GPU in the sum of the computing speeds of the CPU and the GPU.

Specifically, the sum of the computing speeds of the CPU and the GPU is calculated, a ratio of the computing speed of the CPU to this sum is determined as the computing speed proportion of the CPU, and a ratio of the computing speed of the GPU to this sum is determined as the computing speed proportion of the GPU. Thus, the number of particles to be simulated by the CPU is obtained by multiplying the total number of particles to be simulated by the computing speed proportion of the CPU, and the number of particles to be simulated by the GPU is obtained by multiplying the total number of particles to be simulated by the computing speed proportion of the GPU.

As an example, when the computing speeds of the CPU and the GPU are Vc and Vg, respectively, and the total number of particles to be simulated is N, the number of particles simulated on the CPU is N*Vc/(Vc+Vg), and the number of particles simulated on the GPU is N*Vg/(Vc+Vg).

In this embodiment, allocating the computing loads to each of the CPU and the GPU based on the computing speed proportions of the CPU and the GPU allows for full use of the computing resources of both the CPU and the GPU, thereby maximizing the computing speed with the existing CPU and GPU and reducing the dose calculation time.

In an embodiment, as shown in FIG. 3, performing, by the CPU and the GPU, particle simulation according to the CPU and GPU joint dose calculation mode and the dose calculation geometric model to obtain the dose calculation result further includes:

step 302: when the CPU and GPU joint dose calculation mode is a second joint dose calculation mode, performing, by the GPU, particle simulation according to the dose calculation geometric model;
step 304: when the GPU identifies that a number of colliding particles during the simulation exceeds a preset colliding particle threshold, performing, by the CPU, collision processing on the colliding particles to obtain a collision processing result; and
step 306: resuming, by the GPU, the particle simulation based on the collision processing result to obtain the dose calculation result.

The second joint dose calculation mode refers to a mode in which the CPU and the GPU separately complete part of the particle simulation process.

During the GPU simulation calculations, the proportion of time spent on particle collision calculations in the total calculation time is relatively high, reaching up to 30%. However, during the CPU simulation calculations, the proportion of time spent on collision calculations for colliding particles is less than 3%. A primary reason for this difference lies in the computing modes of the CPU and the GPU. In the CPU, instructions executed in each physical core are independent of each other. While in the GPU, every 32 GPU cores need to run instructions synchronously. When computing instructions vary, the cores with different instructions will enter a sleep state, and some cores with the same instructions will run. This results in the GPU having poor capability in handling computational branches and low computing efficiency. For neutron collisions, there are numerous reaction channels for collisions between neutrons and atomic nuclei. The type of collision that each particle undergoes depends on random sampling, which makes the GPU unable to accelerate collision calculations. Consequently, the proportion of time spent on collision calculations by the GPU is significantly higher than that by the CPU. Therefore, the second joint dose calculation mode is provided. In the second joint dose calculation mode, particle simulation is mainly performed by the GPU, and the collision calculation process is transferred to the CPU to be completed. That is, the CPU completes only the collision calculation process of particles, and the GPU completes the particle simulation processes other than the collision calculation process.

Specifically, when the computing capability of the CPU is determined to be poor or medium based on the ratio relationship between the computing speed of the CPU and the computing speed of the GPU, the CPU and GPU joint dose calculation mode is determined to be the second joint dose calculation mode. Specifically, the particle transport process is simulated by the GPU based on the dose calculation geometric model according to the Monte Carlo method. During the particle transport process, when the GPU identifies that a particle is a colliding particle, the GPU stops the simulation process for the particle and stores the colliding particle. When the GPU identifies that the number of colliding particles during the simulation exceeds the preset colliding particle threshold, collision calculation is required. The collision calculation process is transferred to the CPU, and the CPU performs collision processing on the colliding particles to obtain the collision processing result, and sends the collision processing result to the GPU. After obtaining the collision processing result, the GPU resumes simulating the transport process of the colliding particles corresponding to the collision processing result until the simulation of particles of the total number of particles to be simulated is completed, to obtain the dose calculation result. It should be understood that when the CPU performs collision processing on the colliding particles, the GPU can resume simulating other particles, and the two processes do not interfere with each other.

After obtaining the dose calculation result, the GPU transmits the dose calculation result to the CPU, and the CPU takes this dose calculation result as a final dose calculation result.

In this embodiment, when the computing capability of the CPU itself is poor or medium, considering the acceleration characteristic of the CPU in the collision calculation process, the collision calculation process is transferred to the CPU during the particle simulation by the GPU. The use of the CPU for collision calculation allows for full use of the contribution capabilities of both the CPU and the GPU to computing speed, thereby maximizing the computing speed with the existing CPU and GPU.

In an optional implementation of the above embodiment, performing, by the GPU, particle simulation according to the dose calculation geometric model includes: simulating, by the GPU, a particle transport process based on the dose calculation geometric model.

The particle simulation refers to simulating an entire transport process of particles from a radiation source model to a three-dimensional voxel tissue model.

Specifically, by means of a Monte Carlo simulation program, the GPU simulates the transport process of particles from the radiation source model to the three-dimensional voxel tissue model when the target subject is irradiated by a particle beam from the radiation source model, based on the shield model, carrying apparatus model, etc. The particle transport process includes the conditions of the particles in a space between the radiation source model and the three-dimensional voxel tissue model, and the collision trajectories and energy distribution of the particles within the three-dimensional voxel tissue model of the target subject.

Further, in the Monte Carlo simulation program, beam parameters (such as beam energy, intensity, radius, etc.) are defined. By sampling at different irradiation angles, the transport process of the particles from the radiation source model to the three-dimensional voxel tissue model at different irradiation angles is simulated and calculated.

In this embodiment, when the computing capability of the CPU itself is poor or medium, the GPU simulates the particle transport process based on the dose calculation geometric model, so that the computing resources of the GPU can be fully used, thereby improving the dose calculation speed.

In the above optional implementation, the simulation of the particle transport process further includes: a step of identifying colliding particles, which may include: determining, by the GPU, cross-section information of a grid where a particle is located, and determining whether the particle will collide during the transport process based on the cross-section information and a random sampling value; and if a collision will occur, marking, by the GPU, the particle as a colliding particle.

The cross-section information refers to the total macroscopic cross-section of the particle and the material of the grid where the particle is located. The random sampling value refers to a sampled value of a random variable uniformly distributed between 0 and 1.

The GPU determines the material of the current grid where the particle is located during the transport process according to the shield model in the dose calculation geometric model, and calculates the total macroscopic cross-section of the particle and the material of the grid where the particle is located as the cross-section information of the grid where the particle is located.

Since the dose calculation geometric model is not a homogeneous medium, and particle simulation is performed on a grid-by-grid basis, after sampling to obtain a collision distance from the cross-section information of the grid where the particle is located and the random sampling value, it is further necessary to calculate the distance from the particles to a boundary of the grid, i.e., a boundary distance. If the boundary distance is greater than the collision distance, it is identified that the particle will collide when moving to a position at the collision distance, and the particle is marked as a colliding particle.

Specifically, the cross-section information of the grid where the particle is located and the random sampling value are substituted into a collision distance calculation function to obtain the collision distance. The collision distance calculation function can be expressed as follows:

$$L = -\frac{ln\,\xi}{\Sigma_t}$$

where, L represents the collision distance, $\xi$ is a sampled value of a random variable uniformly distributed between 0 and 1, $\Sigma_t$ represents the cross-section information of the grid where the particle is located.

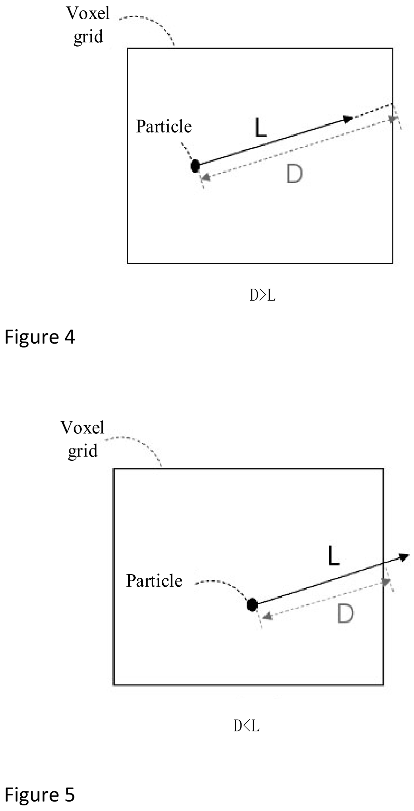

If the boundary distance is greater than the collision distance, it is identified that the particle will collide when moving to a position at the collision distance, and the particle is marked as a colliding particle. When the boundary distance is greater than the collision distance, taking the three-dimensional voxel tissue model as an example, a schematic plan view of the particle in the grid of the three-dimensional voxel tissue model can be shown in FIG. 4, where D represents the boundary distance and L represents the collision distance.

If the boundary distance is less than the collision distance, no collision occurs, and the particle moves to the grid boundary, whereby the particle enters the next grid, and the process returns to the step of identifying colliding particles to re-sample the collision distance and compare it with the boundary distance until the particle exits the grid or is absorbed. When the boundary distance is less than the collision distance, taking the three-dimensional voxel tissue model as an example, a schematic plan view of the particle in the grid of the three-dimensional voxel tissue model can be shown in FIG. 5, where D represents the boundary distance and L represents the collision distance.

In this embodiment, the GPU determines whether a particle will collide during the transport process based on the cross-section information of the grid where the particle is located and the random sampling value, and marks particles that will collide as colliding particles, which can accurately determine whether a particle will collide; and by marking colliding particles, it is easy to distinguish colliding particles from non-colliding particles, which facilitates the rapid transfer of colliding particles to the CPU.

In an embodiment, when the GPU identifies that the number of colliding particles during the simulation exceeds the preset colliding particle threshold, performing, by the CPU, collision processing on the colliding particles includes: when the GPU identifies that the number of colliding particles during the simulation exceeds the preset colliding particle threshold, copying particle information of the colliding particles to the CPU; and performing, by the CPU, collision processing on the colliding particles based on the particle information.

When the GPU identifies that the number of colliding particles during the simulation exceeds the preset colliding particle threshold, it indicates that the number of colliding particles required for the collision calculation process has met the dose calculation requirements, and the GPU copies the particle information of the colliding particles to the CPU. The particle information may include particle type (such as a neutron, a photon, etc.), particle cross-section information, particle nuclide, etc. Collision processing is performed by the CPU on the colliding particles based on the particle information to obtain a particle dose absorbed by the three-dimensional voxel tissue model. The CPU may use a traditional particle collision method for collision calculation.

Further, when the particle type is neutron, the nuclide that collides with the neutron, whether the neutron and the nuclide undergo an absorption reaction, whether the neutron and the nuclide undergo elastic scattering, and whether the neutron and the nuclide undergo inelastic scattering during the collision process are determined based on the neutron information. The neutron dose absorbed by the three-dimensional voxel tissue model is calculated based on the reactions that occur. The absorption reaction refers to a reaction in which neutrons are completely absorbed without producing new neutrons (for example, only photons are produced). When the particle type is photon, whether the photon undergoes coherent scattering (Rayleigh scattering), incoherent scattering (Compton scattering), photoelectric effect, or electron pair effect is determined based on the photon information. The photon dose absorbed by the three-dimensional voxel tissue model is calculated based on the reactions that occur.

In this embodiment, copying the particle information of the colliding particles to the CPU allows the CPU to perform rapid collision processing on the colliding particles based on the particle information.

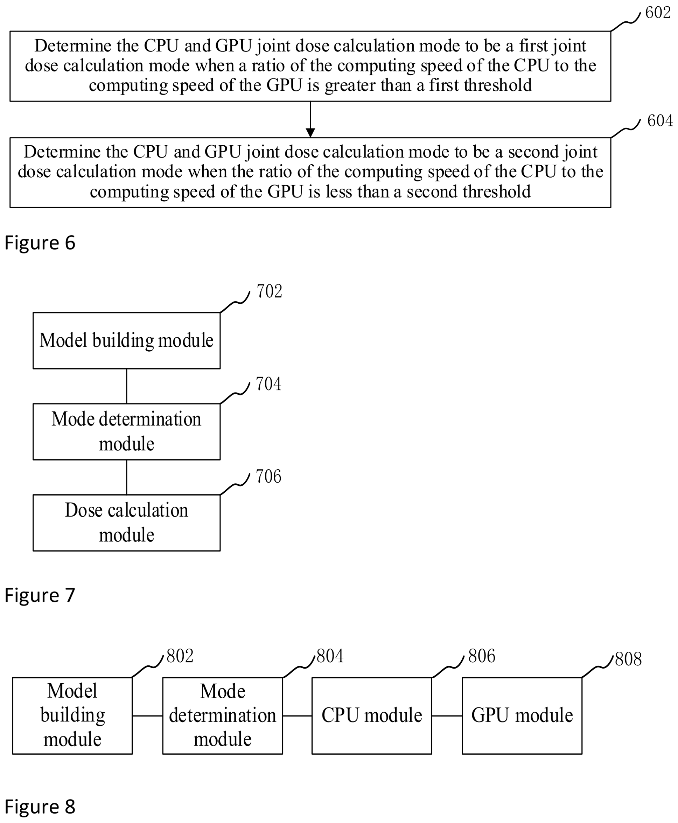

In an embodiment, as shown in FIG. 6, determining the CPU and GPU joint dose calculation mode from the computing speed of the CPU and the computing speed of the GPU includes:

  step 602: determining the CPU and GPU joint dose calculation mode to be a first joint dose calculation mode when a ratio of the computing speed of the CPU to the computing speed of the GPU is greater than a first threshold; and
  step 604: determining the CPU and GPU joint dose calculation mode to be a second joint dose calculation mode when the ratio of the computing speed of the CPU to the computing speed of the GPU is less than a second threshold.

The first threshold and the second threshold are used to determine the level of the computing capability of the CPU. The first threshold is greater than the second threshold.

Specifically, the computing capability of the CPU is determined based on the ratio of the computing speed of the CPU to the computing speed of the GPU. When the ratio is greater than the first threshold, it indicates that the computing capability of the CPU is high, and the CPU and GPU joint dose calculation mode can be determined to be the first joint dose calculation mode. When the ratio is less than the second threshold, it indicates that the computing capability of the CPU is poor, and the CPU and GPU joint dose calculation mode can be determined to be the second joint dose calculation mode.

Further, the first threshold is 0.3, and the second threshold is 0.15. The first threshold and the second threshold can be obtained from a large number of dose calculation experiments. Thus, during actual dose calculation, the dose calculation system can flexibly determine the CPU and GPU joint dose calculation mode based on the ratio of the computing speed of the CPU to the computing speed of the GPU.

In this embodiment, the level of the computing capability of the CPU is determined based on the ratio of the computing speed of the CPU to the computing speed of the GPU, so that the CPU and GPU joint dose calculation mode is determined based on the computing capability of the CPU itself, enabling the allocation of computing loads based on the actual computing capabilities of the CPU and the GPU, and thus maximizing the computing speed with the CPU and the GPU.

Further, determining the CPU and GPU joint dose calculation mode from the computing speed of the CPU and the computing speed of the GPU further includes: when the ratio of the computing speed of the CPU to the computing speed of the GPU is less than or equal to the first threshold and greater than or equal to the second threshold, determining the CPU and GPU joint dose calculation mode to be either the first joint dose calculation mode or the second joint dose calculation mode.

When the ratio of the computing speed of the CPU to the computing speed of the GPU falls between the second threshold and the first threshold, it indicates that the computing capability of the CPU is medium, and either of the first joint dose calculation mode and the second joint dose calculation mode can be selected as the CPU and GPU joint dose calculation mode.

In this embodiment, when the ratio of the computing speed of the CPU to the computing speed of the GPU is less than or equal to the first threshold and greater than or equal to the second threshold, either the first joint dose calculation mode or the second joint dose calculation mode can be selected, enabling flexible determination of the CPU and GPU joint dose calculation mode.

It should be understood that although the steps in the flowcharts involved in the above embodiments are shown in sequence as indicated by the arrows, these steps are not necessarily performed in sequence in the order indicated by the arrows. Unless explicitly described herein, the performance of these steps is not limited to a strict order, instead, the steps may be performed in another order. Furthermore, at least some of the steps in the flowcharts involved in the above embodiments may include a plurality of steps or stages. These steps or stages are not necessarily completed at the same time, but may be performed at different moments. These steps or stages are not necessarily performed in sequence, but may be performed in turn or alternately with other steps or at least some of steps or stages in other steps.

Based on the same inventive concept, the embodiments of the present application further provide a control apparatus for a dose calculation system, which is configured to implement the control method for a dose calculation system involved above. The implementation solutions for solving problems provided by this apparatus are similar to those described in the method described above. Therefore, the definitions above on the control method for a dose calculation system can be referred to for the specific definitions in the following one or more embodiments of the control apparatus for a dose calculation system, which will not be repeated here.

In an embodiment, as shown in FIG. 7, a control apparatus for a dose calculation system is provided. The dose calculation system includes a CPU and a GPU; and the control apparatus for a dose calculation system includes: a model building module 702, a mode determination module 704, and a dose calculation module 706.

The model building module 702 is configured to build a dose calculation geometric model.

The mode determination module 704 is configured to determine a CPU and GPU joint dose calculation mode from a computing speed of the CPU and a computing speed of the GPU.

The dose calculation module 706 is configured to control the CPU and the GPU to perform particle simulation according to the CPU and GPU joint dose calculation mode and the dose calculation geometric model to obtain a dose calculation result.

In an embodiment, the dose calculation module 706 is further configured to: when the CPU and GPU joint dose calculation mode is a first joint dose calculation mode, determine a respective number of particles to be simulated by the CPU and the GPU from the computing speed of the CPU, the computing speed of the GPU, and a total number of particles to be simulated; control the CPU to simulate the respective number of particles to be simulated according to the dose calculation geometric model to obtain a CPU dose calculation result; control the GPU to simulate the respective number of particles to be simulated according to the dose calculation geometric model to obtain a GPU dose calculation result; and sum the CPU dose calculation result and the GPU dose calculation result to obtain the dose calculation result.

In an embodiment, the dose calculation module 706 is further configured to: determine a computing speed proportion of the CPU and a computing speed proportion of the GPU from the computing speed of the CPU and the computing speed of the GPU; determine the number of particles to be simulated by the CPU from the computing speed proportion of the CPU and the total number of particles to be simulated; and determine the number of particles to be simulated by the CPU from the computing speed proportion of the GPU and the total number of particles to be simulated.

In an embodiment, the dose calculation module 706 is further configured to: when the CPU and GPU joint dose calculation mode is a second joint dose calculation mode, control the GPU to perform particle simulation according to the dose calculation geometric model; when the GPU identifies that a number of colliding particles during the simulation exceeds a preset colliding particle threshold, control the CPU to perform collision processing on the colliding particles to obtain a collision processing result; and control the GPU to resume the particle simulation based on the collision processing result to obtain the dose calculation result.

In an embodiment, the dose calculation module 706 is further configured to control the GPU to simulate a particle transport process based on the dose calculation geometric model.

In an embodiment, the dose calculation module 706 is further configured to: control the GPU to determine cross-section information of a grid where a particle is located, and determine whether the particle will collide during the transport process based on the cross-section information and a random sampling value; and if a collision will occur, control the GPU to mark the particle as a colliding particle.

In an embodiment, the dose calculation module 706 is further configured to: when the GPU identifies that the number of colliding particles during the simulation exceeds the preset colliding particle threshold, control the GPU to copy particle information of the colliding particles to the CPU; and control the CPU to perform collision processing on the colliding particles based on the particle information.

In an embodiment, the mode determination module 704 is further configured to:
determine the CPU and GPU joint dose calculation mode to be the first joint dose calculation mode when a ratio of the computing speed of the CPU to the computing speed of the GPU is greater than a first threshold; and determine the CPU and GPU joint dose calculation mode to be the second joint dose calculation mode when the ratio of the computing speed of the CPU to the computing speed of the GPU is less than a second threshold; where the first threshold is greater than the second threshold.

In an embodiment, the mode determination module 704 is further configured to: when the ratio of the computing speed of the CPU to the computing speed of the GPU is less than or equal to the first threshold and greater than or equal to the second threshold, determine the CPU and GPU joint dose calculation mode to be either the first joint dose calculation mode or the second joint dose calculation mode.

In an embodiment, the first threshold is 0.3, and the second threshold is 0.15.

All modules in the control apparatus for a dose calculation system as described above can be fully or partially implemented through software, hardware, or a combination thereof. Each of the above modules can be embedded in or independent of the processor in the computer device in the form of hardware, or stored in the memory of the computer device in the form of software, so that the processor can invoke them to execute the operations corresponding to each of the above modules.

In an embodiment, as shown in FIG. 8, a dose calculation system is provided. The dose calculation system includes: a model building module 802, a mode determination module 804, a CPU module 806, and a GPU module 808.

The model building module 802 is configured to build a dose calculation geometric model.

The mode determination module 804 is configured to determine a CPU and GPU joint dose calculation mode from a computing speed of a CPU and a computing speed of a GPU.

The CPU module 806 is configured to perform at least part of dose calculation according to the CPU and GPU joint dose calculation mode and the dose calculation geometric model.

The GPU module 808 is configured to perform at least part of the dose calculation according to the CPU and GPU joint dose calculation mode and the dose calculation geometric model.

In an embodiment, the dose calculation system further includes:
a particle allocation module configured to, when the CPU and GPU joint dose calculation mode is a first joint dose calculation mode, determine a respective number of particles to be simulated by the CPU and the GPU from the computing speed of the CPU, the computing speed of the GPU, and a total number of particles to be simulated.

The CPU module 806 is configured to simulate the respective number of particles to be simulated according to the dose calculation geometric model to obtain a CPU dose calculation result.

The GPU module 808 is configured to simulate the respective number of particles to be simulated according to the dose calculation geometric model to obtain a GPU dose calculation result.

A result summing module is configured to sum the CPU dose calculation result and the GPU dose calculation result to obtain the dose calculation result.

In an embodiment, the particle allocation module is further configured to: determine a computing speed proportion of the CPU and a computing speed proportion of the GPU from the computing speed of the CPU and the computing speed of the GPU; determine the number of particles to be simulated by the CPU from the computing speed proportion of the CPU and the total number of particles to be simulated; and determine the number of particles to be simulated by the CPU from the computing speed proportion of the GPU and the total number of particles to be simulated.

In an embodiment, the GPU module 808 is further configured to: when the CPU and GPU joint dose calculation mode is a second joint dose calculation mode, perform particle simulation according to the dose calculation geometric model; and when identifying that a number of colliding particles during the simulation exceeds a preset colliding particle threshold, use the CPU module 806 to perform collision processing on the colliding particles to obtain a collision processing result. The GPU module 808 is further configured to resume the particle simulation based on the collision processing result to obtain the dose calculation result.

In an embodiment, the GPU module 808 is further configured to simulate a particle transport process based on the dose calculation geometric model.

In an embodiment, the GPU module 808 is further configured to: determine cross-section information of a grid where a particle is located, and determine whether the particle will collide during the transport process based on the cross-section information and a random sampling value; and if a collision will occur, mark the particle as a colliding particle.

In an embodiment, the GPU module 808 is further configured to: when identifying that the number of colliding particles during the simulation exceeds the preset colliding particle threshold, copy particle information of the colliding particles to the CPU module 806. The CPU module 806 is further configured to perform collision processing on the colliding particles based on the particle information.

In an embodiment, the mode determination module 804 is further configured to:
determine the CPU and GPU joint dose calculation mode to be the first joint dose calculation mode when a ratio of the computing speed of the CPU to the computing speed of the GPU is greater than a first threshold; and determine the CPU and GPU joint dose calculation mode to be the second joint dose calculation mode when the ratio of the computing speed of the CPU to the computing speed of the GPU is less than a second threshold; where the first threshold is greater than the second threshold.

In an embodiment, the mode determination module 804 is further configured to: when the ratio of the computing speed of the CPU to the computing speed of the GPU is less than or equal to the first threshold and greater than or equal to the second threshold, determine the CPU and GPU joint dose calculation mode to be either the first joint dose calculation mode or the second joint dose calculation mode.

In an embodiment, the first threshold is 0.3, and the second threshold is 0.15.

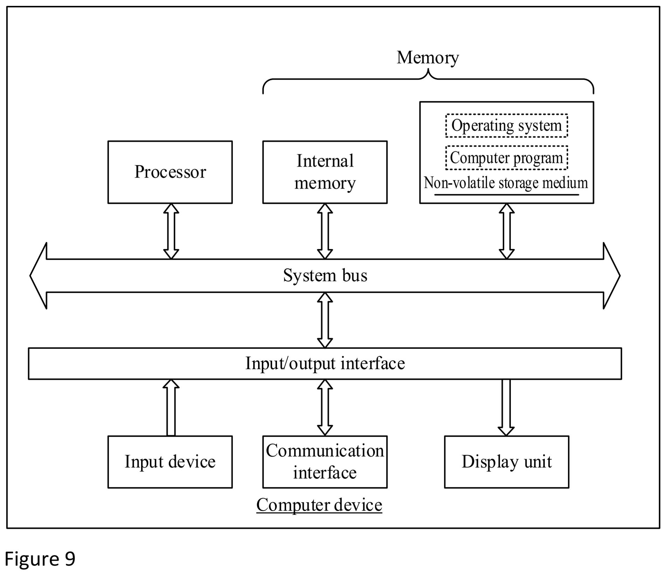

In an embodiment, a computer device is provided, which may be a terminal. A diagram of an internal structure of the computer device may be as shown in FIG. 9. The computer device includes a processor, a memory, an input/output interface, a communication interface, a display unit, and an input device. The processor, the memory, and the input/output interface are connected via a system bus, and the communication interface, the display unit, and the input device are connected to the system bus via the input/output interface. The processor of the computer device is configured to provide computing and control capabilities. The memory of the computer device includes a non-volatile storage medium and an internal memory. The non-volatile storage medium stores an operating system and a computer program. The internal memory provides an environment for the operation of the operating system and the computer program in the non-volatile storage medium. The input/output interface of the computer device is configured to exchange information between the processor and external devices. The communication interface of the computer device is configured to communicate with external terminals in a wired or wireless manner. The wireless manner can be realized by means of WIFI, mobile cellular network, NFC (Near Field Communication) or other technologies. The computer program, when executed by the processor, causes a control method for a dose calculation system to be implemented. The display unit of the computer device is configured to form a visually visible image, and can be a display screen, a projection apparatus or a virtual reality imaging apparatus. The display screen can be a liquid crystal display or an electronic ink display. The input device of the computer device can be a touch layer covered on the display screen, or keys, a trackball or a touchpad arranged on a housing of the computer device, or an external keyboard, touchpad or mouse, etc.

Those skilled in the art will understand that the structure shown in FIG. 9 is merely a block diagram of part of the structure related to the solution of the present application, and does not constitute a limitation on the computer device to which the solution of the present application is applied. A specific computer device may include more or fewer components than those shown in the figure, or combine some components, or have a different arrangement of components.

In an embodiment, a computer device is provided, which includes a memory and a processor, where a computer program is stored in the memory, and the processor, when executing the computer program, causes the steps in each of the above method embodiments to be implemented.

In an embodiment, a computer-readable storage medium is provided, on which a computer program is stored, where the computer program, when executed by a processor, causes the steps in each of the above method embodiments to be implemented.

In an embodiment, a computer program product is provided, which includes a computer program which, when executed by a processor, causes the steps in each of the above method embodiments to be implemented.

Those of ordinary skill in the art will understand that all or part of the processes in the methods of the above embodiments can be completed by instructing relevant hardware through a computer program. The computer program can be stored in a non-volatile computer-readable storage medium. The computer program, when executed, can include the processes of the above method embodiments. Any reference to the memory, the database or other media used in the embodiments provided in the present application may include at least one of non-volatile and volatile memories. The non-volatile memory may include a read-only memory (ROM), a magnetic tape, a floppy disk, a flash memory, an optical memory, a high-density embedded non-volatile memory, a resistive random access memory (ReRAM), a magnetoresistive random access memory (MRAM), a ferroelectric random access memory (FRAM), a phase change memory (PCM), a graphene memory, and the like. The volatile memory may include a random access memory (RAM), an external cache memory, and the like. By way of illustration rather than limitation, the RAM may be in various forms, such as a static random access memory (SRAM) or a dynamic random access memory (DRAM). The database involved in the embodiments provided in the present application may include at least one of a relational database and a non-relational database. The non-relational database may include, but is not limited to, a blockchain-based distributed database, and the like. The processor involved in the embodiments provided in the present application may be but is not limited to a general-purpose processor, a central processing unit, a graphics processor, a digital signal processor, a programmable logic device, a quantum computing-based data processing logic device, and the like.

The technical features of the above embodiments may be combined arbitrarily. For the purpose of brevity of description, all the possible combinations of the technical features in the above embodiments are not described. However, as long as there is no contradiction between the combinations of these technical features, they shall all fall within the scope of the specification.

The embodiments described above merely illustrate several implementations of the present application and are described relatively specifically and in detail, but should not be construed as limiting the patent scope of the present application. It should be noted that several variations and improvements may also be made by those of ordinary skill in the art without departing from the concept of the present application, and those modifications and improvements shall all fall within the scope of protection of the present application. Therefore, the scope of protection of the present application shall be subject to the appended claims.

**Claims**

1. A control method for a dose calculation system comprising a CPU and a GPU, the method comprising:

   building a dose calculation geometric model;
   determining a CPU and GPU joint dose calculation mode from a computing speed of the CPU and a computing speed of the GPU; and
   performing, by the CPU and the GPU, particle simulation according to the CPU and GPU joint dose calculation mode and the dose calculation geometric model to obtain a dose calculation result.

2. The control method according to claim 1, wherein performing, by the CPU and the GPU, particle simulation according to the CPU and GPU joint dose calculation mode and the dose calculation geometric model to obtain the dose calculation result comprises:

   when the CPU and GPU joint dose calculation mode is a first joint dose calculation mode,
   determining a respective number of particles to be simulated by the CPU and the GPU from the computing speed of the CPU, the computing speed of the GPU, and a total number of particles to be simulated;
   simulating, respectively by the CPU and the GPU, the respective number of particles to be simulated according to the dose calculation geometric model to obtain a CPU dose calculation result and a GPU dose calculation result; and
   summing the CPU dose calculation result and the GPU dose calculation result to obtain the dose calculation result.

3. The control method according to claim 2, wherein determining the respective number of particles to be simulated by the CPU and the GPU from the computing speed of the CPU, the computing speed of the GPU, and the total number of particles to be simulated comprises:

   determining a computing speed proportion of the CPU and a computing speed proportion of the GPU from the computing speed of the CPU and the computing speed of the GPU;
   determining the number of particles to be simulated by the CPU from the computing speed proportion of the CPU and the total number of particles to be simulated; and
   determining the number of particles to be simulated by the CPU from the computing speed proportion of the GPU and the total number of particles to be simulated.

**4.** The control method according to claim 1, wherein performing, by the CPU and the GPU, particle simulation according to the CPU and GPU joint dose calculation mode and the dose calculation geometric model to obtain the dose calculation result further comprises:

when the CPU and GPU joint dose calculation mode is a second joint dose calculation mode, performing, by the GPU, particle simulation according to the dose calculation geometric model; when the GPU identifies that a number of colliding particles during the simulation exceeds a preset colliding particle threshold, performing, by the CPU, collision processing on the colliding particles to obtain a collision processing result; and

resuming, by the GPU, the particle simulation based on the collision processing result to obtain the dose calculation result.

**5.** The control method according to claim 1, wherein determining the CPU and GPU joint dose calculation mode from the computing speed of the CPU and the computing speed of the GPU comprises:

determining the CPU and GPU joint dose calculation mode to be a first joint dose calculation mode when a ratio of the computing speed of the CPU to the computing speed of the GPU is greater than a first threshold; and determining the CPU and GPU joint dose calculation mode to be a second joint dose calculation mode when the ratio of the computing speed of the CPU to the computing speed of the GPU is less than a second threshold; wherein the first threshold is greater than the second threshold.

**6.** The control method according to claim 8, wherein determining the CPU and GPU joint dose calculation mode from the computing speed of the CPU and the computing speed of the GPU further comprises:

when the ratio of the computing speed of the CPU to the computing speed of the GPU is less than or equal to the first threshold and greater than or equal to the second threshold, determining the CPU and GPU joint dose calculation mode to be either the first joint dose calculation mode or the second joint dose calculation mode.

**7.** A control apparatus for a dose calculation system comprising a CPU and a GPU, the control apparatus for a dose calculation system comprising:

a model building module configured to build a dose calculation geometric model;

a mode determination module configured to determine a CPU and GPU joint dose calculation mode from a computing speed of the CPU and a computing speed of the GPU; and
a dose calculation module configured to control the CPU and the GPU to perform particle simulation according to the CPU and GPU joint dose calculation mode and the dose calculation geometric model to obtain a dose calculation result.

**8.** The control apparatus according to claim 7, wherein the dose calculation module is further configured to: when the CPU and GPU joint dose calculation mode is a first joint dose calculation mode, determine a respective number of particles to be simulated by the CPU and the GPU from the computing speed of the CPU, the computing speed of the GPU, and a total number of particles to be simulated; control the CPU to simulate the respective number of particles to be simulated according to the dose calculation geometric model to obtain a CPU dose calculation result; control the GPU to simulate the respective number of particles to be simulated according to the dose calculation geometric model to obtain a GPU dose calculation result; and sum the CPU dose calculation result and the GPU dose calculation result to obtain the dose calculation result.

**9.** The control apparatus according to claim 8, wherein the dose calculation module is further configured to: determine a computing speed proportion of the CPU and a computing speed proportion of the GPU from the computing speed of the CPU and the computing speed of the GPU; determine the number of particles to be simulated by the CPU from the computing speed proportion of the CPU and the total number of particles to be simulated; and determine the number of particles to be simulated by the CPU from the computing speed proportion of the GPU and the total number of particles to be simulated.

**10.** The control apparatus according to claim 7, wherein the dose calculation module is further configured to: when the CPU and GPU joint dose calculation mode is a second joint dose calculation mode, control the GPU to perform particle simulation according to the dose calculation geometric model; when the GPU identifies that a number of colliding particles during the simulation exceeds a preset colliding particle threshold, control the CPU to perform collision processing on the colliding particles to obtain a collision processing result; and control the GPU to resume the particle simulation based on the collision processing result to obtain the dose calculation result.

**11.** A dose calculation system, comprising:

a model building module configured to build a dose calculation geometric model;
a mode determination module configured to determine a CPU and GPU joint dose calculation mode from a computing speed of a CPU and a computing speed of a GPU;
a CPU module configured to perform at least part of dose calculation according to the CPU and GPU joint dose calculation mode and the dose calculation geometric model; and
a GPU module configured to perform at least part of the dose calculation according to the CPU and GPU joint dose calculation mode and the dose calculation geometric model.

12. A computer device, comprising a memory and a processor, wherein the memory stores a computer program, and the processor, when executing the computer program, causes the following steps to be implemented:

building a dose calculation geometric model;
determining a CPU and GPU joint dose calculation mode from a computing speed of a CPU and a computing speed of a GPU; and
performing, by the CPU and the GPU, particle simulation according to the CPU and GPU joint dose calculation mode and the dose calculation geometric model to obtain a dose calculation result.

13. A computer-readable storage medium, wherein the computer-readable storage medium stores a computer program which, when executed by a processor, causes the following steps to be implemented:

building a dose calculation geometric model;
determining a CPU and GPU joint dose calculation mode from a computing speed of a CPU and a computing speed of a GPU; and
performing, by the CPU and the GPU, particle simulation according to the CPU and GPU joint dose calculation mode and the dose calculation geometric model to obtain a dose calculation result.

14. A computer program product, comprising a computer program which, when executed by a processor, causes the following steps to be implemented:

building a dose calculation geometric model;
determining a CPU and GPU joint dose calculation mode from a computing speed of a CPU and a computing speed of a GPU; and
performing, by the CPU and the GPU, particle simulation according to the CPU and GPU joint dose calculation mode and the dose calculation geometric model to obtain a dose calculation result.

102

Build a dose calculation geometric model

104

Determine a CPU and GPU joint dose calculation mode from a computing speed of a CPU and a computing speed of a GPU

106

Perform, by the CPU and the GPU, particle simulation according to the CPU and GPU joint dose calculation mode and the dose calculation geometric model to obtain a dose calculation result

Figure 1

| When the CPU and GPU joint dose calculation mode is a first joint dose calculation mode, determine a respective number of particles to be simulated by the CPU and the GPU from the computing speed of the CPU, the computing speed of the GPU, and a total number of particles to be simulated | 202 |

| Simulate, respectively by the CPU and the GPU, the respective number of particles to be simulated according to the dose calculation geometric model to obtain a CPU dose calculation result and a GPU dose calculation result | 204 |

| Sum the CPU dose calculation result and the GPU dose calculation result to obtain the dose calculation result | 206 |

Figure 2

| When the CPU and GPU joint dose calculation mode is a second joint dose calculation mode, perform, by the GPU, particle simulation according to the dose calculation geometric model | 302 |

| When the GPU identifies that a number of colliding particles during the simulation exceeds a preset colliding particle threshold, perform, by the CPU, collision processing on the colliding particles to obtain a collision processing result | 304 |

| Resume, by the GPU, the particle simulation based on the collision processing result to obtain the dose calculation result | 306 |

Figure 3

Figure 4

Figure 5

602

Determine the CPU and GPU joint dose calculation mode to be a first joint dose calculation mode when a ratio of the computing speed of the CPU to the computing speed of the GPU is greater than a first threshold

604

Determine the CPU and GPU joint dose calculation mode to be a second joint dose calculation mode when the ratio of the computing speed of the CPU to the computing speed of the GPU is less than a second threshold

Figure 6

702

Model building module

704

Mode determination module

706

Dose calculation module

Figure 7

802

Model building module

804

Mode determination module

806

CPU module

808

GPU module

Figure 8

Figure 9

# EP 4 703 880 A1

## INTERNATIONAL SEARCH REPORT

International application No.

**PCT/CN2024/089786**

### A. CLASSIFICATION OF SUBJECT MATTER

G06F9/50(2006.01)i; A61N5/10(2006.01)n

According to International Patent Classification (IPC) or to both national classification and IPC

### B. FIELDS SEARCHED

Minimum documentation searched (classification system followed by classification symbols)

IPC: G06F, A61N

Documentation searched other than minimum documentation to the extent that such documents are included in the fields searched

Electronic data base consulted during the international search (name of data base and, where practicable, search terms used)

VEN, CNABS, CNTXT, WOTXT, USTXT, CNKI, IEEE: 比值, 比例, 运算, 计算, 时间, 速度, 并行, 策略, 阈值, 粒子, 碰撞, 数, ratio, proportion, calculation, operation, time, speed, parallel, strategy, threshold, particle, collision, number, CPU, GPU

### C. DOCUMENTS CONSIDERED TO BE RELEVANT

| Category* | Citation of document, with indication, where appropriate, of the relevant passages | Relevant to claim No. |
|---|---|---|
| X | CN 109871352 A (BEIJING SOGOU TECHNOLOGY DEVELOPMENT CO., LTD.) 11 June 2019 (2019-06-11) description, paragraphs 0018-0033 and 0073-0076 | 1-3, 5-9, 11-14 |
| X | CN 105468439 A (EAST CHINA NORMAL UNIVERSITY) 06 April 2016 (2016-04-06) description, paragraphs 0029-0037 | 1-3, 5-9, 11-14 |
| A | CN 101954148 A (SICHUAN UNIVERSITY) 26 January 2011 (2011-01-26) entire document | 1-14 |
| A | CN 103955567 A (INSTITUTE OF MODERN PHYSICS, CHINESE ACADEMY OF SCIENCES) 30 July 2014 (2014-07-30) entire document | 1-14 |

☐ Further documents are listed in the continuation of Box C.   ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **25 June 2024** | **16 July 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

22

**INTERNATIONAL SEARCH REPORT**
Information on patent family members

| International application No. |
| PCT/CN2024/089786 |

| Patent document cited in search report | | | Publication date (day/month/year) | Patent family member(s) | Publication date (day/month/year) |
|---|---|---|---|---|---|
| CN | 109871352 | A | 11 June 2019 | None | |
| CN | 105468439 | A | 06 April 2016 | None | |
| CN | 101954148 | A | 26 January 2011 | None | |
| CN | 103955567 | A | 30 July 2014 | None | |

Form PCT/ISA/210 (patent family annex) (July 2022)